# EUROPEAN PATENT APPLICATION

(11) **EP 4 194 472 A1**
(43) Date of publication of application: **14.06.2023**
(21) Application number: 21866033.0
(22) Date of filing: 09.09.2021
(51) Int. Cl.: C07K 19/00, C12N 5/10, C12N 15/62, A61K 35/17

(54) **CHIMERIC ANTIGEN RECEPTOR COMPRISING NOVEL CO-STIMULATORY DOMAIN AND USE THEREOF**

(30) Priority: 10.09.2020 CN 202010947407
(71) Applicant: Bioheng Therapeutics Limited, Grand Cayman KY1-1209 (KY)
(72) Inventor: ZHOU, Yali, Nanjing, Jiangsu 210061 (CN); CHEN, Gong, Nanjing, Jiangsu 210061 (CN); JIANG, Xiaoyan, Nanjing, Jiangsu 210061 (CN); REN, Jiangtao, Nanjing, Jiangsu 210061 (CN); HE, Xiaohong, Nanjing, Jiangsu 210000 (CN); WANG, Yanbin, Nanjing, Jiangsu 210000 (CN); HAN, Lu, Nanjing, Jiangsu 210061 (CN)
(74) Representative: Rimini, Rebecca
(86) International application number: PCT/CN2021/117402
(87) International publication number: WO 2022/052981

(57) **Abstract**

Provided is a chimeric antigen receptor, comprising a ligand-binding domain, a transmembrane domain, a co-stimulatory domain, and an intracellular signaling domain. The co-stimulatory domain comprises an intracellular region of an NK-activated receptor or a ligand thereof. Also provided are an engineered immune cell comprising the chimeric antigen receptor and a use thereof in treatment of diseases, such as cancers, autoimmune diseases, and infections.

## Description

### Technical Field

The present disclosure belongs to the field of immunotherapy. More specifically, the present disclosure relates to a chimeric antigen receptor comprising a novel co-stimulatory domain, and an engineered immune cell comprising such chimeric antigen receptor and use thereof.

### Background Art

In recent years, cancer immunotherapy technology has developed rapidly, especially chimeric antigen receptor T cell (CAR-T)-related immunotherapy has achieved excellent clinical results in the treatment of hematological tumors. In CAR-T cell immunotherapy, T cells were genetically modified in vitro so that they can recognize tumor antigens, and after amplified to a certain number, they are reinfused back into the patient's body to kill cancer cells, thereby achieving the purpose of treating tumors.

At present, with the development of technology, four generations of different CAR structures have emerged. The intracellular signaling domain of the first-generation CAR only comprises a primary signaling domain, such as CD3ζ, so CAR-carrying cells (such as CAR-T cells) have poor activity and short survival time in vivo. The second-generation CARs comprise a co-stimulatory domain, such as CD28 or 4-1 BB, which enables cells to proliferate continuously and enhance anti-tumor activity. The third-generation CARs comprise two co-stimulatory domains (such as CD28+4-1 BB), and the fourth-generation CARs have cytokines or co-stimulatory ligands to further enhance T cell responses, or have suicide genes to make CAR-T cells self-destruct when needed. Most of the current clinical research still uses the second-generation CAR structure.

However, there are still some problems in the clinical application of CAR-T cell therapy, for example, a large number of tumor recurrences in the treatment of hematological tumors, and low response rate in the treatment of solid tumors, etc. These may be caused by complex tumor microenvironment, CAR-T cell depletion and other factors.

Therefore, there is still a need to improve the existing CAR T cell therapy to promote the proliferation of CAR T cells in vivo, resist the immunosuppressive effect of the tumor microenvironment, and improve the overall therapeutic effect of CAR T cell therapy on tumors.

### Summary

Therefore, in a first aspect, the present disclosure provides a chimeric antigen receptor comprising a ligand binding domain, a transmembrane domain, a co-stimulatory domain and an intracellular signaling domain, wherein the co-stimulatory domain comprises an intracellular region of an NK activating receptor or a ligand thereof.

In an embodiment, the NK activating receptor is selected from the group consisting of 2B4, DNAM-1 and LFA-1, more preferably 2B4.

In an embodiment, the ligand of the NK activating receptor is selected from the group consisting of CD48, CD112, CD155, ICAM1, ICAM2 and ICAM3, more preferably CD155 and ICAM-3.

In a preferred embodiment, the chimeric antigen receptor of the present disclosure comprises a co-stimulatory domain" which comprises an intracellular region of a protein selected from the group consisting of: CD155, ICAM3 and 2B4. In an embodiment, the CD155 intracellular region has at least 90%, 95%, 97% or 99% or 100% sequence identity to the amino acid sequence represented by SEQ ID NO: 29; the ICAM3 intracellular region has at least 90%, 95%, 97% or 99% or 100% sequence identity to the amino acid sequence represented by SEQ ID NO: 27; and the 2B4 intracellular region has at least 90%, 95%, 97% or 99% or 100% sequence identity to the amino acid sequence represented by SEQ ID NO: 31.

In an embodiment, the co-stimulatory domain further comprises a signaling domain of a protein selected from the group consisting of: TLR1, TLR2, TLR3, TLR4, TLR5, TLR6, TLR7, TLR8, TLR9, TLR10, CARD11, CD2, CD7, CD8, CD27, CD28, CD30, CD40, CD83, CD134 (OX40), CD137 (4-1BB), CD270 (HVEM), CD272 (BTLA), CD276 (B7-H3), CD278 (ICOS), CD357 (GITR), DAP10, DAP12, LAT, NKG2C, SLP76, PD-1, LIGHT, TRIM, CD94, LTB, ZAP70, and a combination thereof. Preferably, the co-stimulatory domain further comprises a signaling domain of CD27, CD28, CD134, CD137 or CD278 or a combination thereof, more preferably further comprises a signaling domain of CD28 and/or CD137.

In an embodiment, the ligand binding domain is an antibody or an antigen binding portion thereof.

In an embodiment, the ligand binding domain is selected from the group consisting of an immunoglobulin molecule, Fab, Fab', F(ab')2, Fv fragment, scFv antibody fragment, heavy chain antibody, linear antibody, sdAb or nanobody.

In an embodiment, the ligand binding domain binds to one or more targets selected from the group consisting of: CD2, CD3, CD4, CD5, CD7, CD8, CD14, CD15, CD46, CD70, TSHR, CD19, CD123, CD22, BAFF-R, CD30, CD171, CS-1, CLL-1, CD33, EGFRvIII, GD2, GD3, BCMA, GPRC5D, Tn Ag, PSMA, ROR1, FLT3, FAP, TAG72, CD38, CD44v6, CEA, EPCAM, B7H3, KIT, IL-13Ra2, mesothelin, IL-1 1Ra, PSCA, PRSS21, VEGFR2, LewisY, CD24, PDGFR-β, SSEA-4, CD20, AFP, Folate receptor α, ERBB2 (Her2/neu), MUC1, EGFR, CS1, CD138, NCAM, Claudin18.2, Prostase, PAP, ELF2M, Ephrin B2, IGF-I receptor, CAlX, LMP2, gploo, bcr-abl, tyrosinase, EphA2, Fucosyl GM1, sLe, GM3, TGS5, HMWMAA, o-acetyl-GD2, Folate receptor β, TEM1/CD248, TEM7R, CLDN6, GPRC5D, CXORF61, CD97, CD179a, ALK, polysialic acid, PLAC1, GloboH, NY-BR-1, UPK2, HAVCR1, ADRB3, PANX3, GPR20, LY6K, OR51E2, TARP, WT1, NY-ESO-1, LAGE-1a, MAGE-A1, legumain, HPV E6, E7, MAGE A1, ETV6-AML, sperm protein 17, XAGE1, Tie 2, MAD-CT-1, MAD-CT-2, Fos associated antigen 1, p53, p53 mutant, prostate specific protein, survivin and telomerase, PCTA-1/Galectin 8, MelanA/MART1, Ras mutant, hTERT, sarcoma translocation breakpoint, ML-IAP, ERG (TMPRSS2 ETS fusion gene), NA17, PAX3, androgen receptor, Cyclin B1, MYCN, RhoC, TRP-2, CYP1B 1, BORIS, SART3, PAX5, OY-TES 1, LCK, AKAP-4, SSX2, RAGE-1, human telomerase reverse transcriptase, RU1, RU2, intestinal tract carboxylesterase, mut hsp70-2, CD79a, CD79b, CD72, LAIR1, FCAR, LILRA2, CD300LF, CLEC12A, BST2, EMR2, LY75, GPC3, FCRL5, IGLL1, PD1, PDL1, PDL2, TGF β, APRIL, NKG2D, NKG2D ligand, and/or pathogen-specific antigen, biotinylated molecule, molecule expressed by HIV, HCV, HBV, and/or other pathogens; and/or neo-epitope or neoantigen. Preferably, the ligand binding domain binds to a target selected from the group consisting of CD19, CD20, CD22, CD30, CD33, CD38, CD123, CD138, CD171, MUC1, AFP, Folate receptor α, CEA, PSCA, PSMA, Her2, EGFR, IL13Ra2, GD2, NKG2D, EGFRvIII, CS1, BCMA, mesothelin, and any combination thereof.

In an embodiment, the transmembrane domain is a transmembrane domain of a protein selected from the group consisting of: TCR α chain, TCR β chain, TCR γ chain, TCR δ chain, CD3 ζ subunit, CD3 ε subunit, CD3 γ subunit, CD3 δ subunit, CD45, CD4, CD5, CD8 α, CD9, CD16, CD22, CD33, CD28, CD37, CD64, CD80, CD86, CD134, CD137, and CD154.

In an embodiment, the intracellular signaling domain is a signaling domain of a protein selected from the group consisting of: FcR γ, FcR β, CD3 γ, CD3 δ, CD3 ε, CD3 ζ, CD22, CD79a, CD79b, and CD66d. Preferably, the intracellular signaling domain is a signaling domain containing CD3 ζ.

The present disclosure further provides a nucleic acid molecule encoding the novel chimeric antigen receptor as described above and a vector comprising the nucleic acid molecule.

In a second aspect, the present disclosure further provides an engineered immune cell comprising the novel chimeric antigen receptor, nucleic acid molecule or vector as described above.

In an embodiment, the endogenous expression of the NK activating receptor or ligand corresponding to the co-stimulatory domain in the engineered immune cell is suppressed or silenced. In a preferred embodiment, the NK activating receptor is 2B4 and the ligand of the NK activating receptor is CD155 or ICAM3.

In an embodiment, the engineered immune cell further comprises suppressed or silenced expression of at least one gene selected from the group consisting of: CD52, GR, dCK, TCR/CD3 genes (e.g. TRAC, TRBC, CD3γ, CD3δ, CD3ε, CD3ζ), MHC related genes (HLA-A, HLA-B, HLA-C, B2M, HLA-DPA, HLA-DQ, HLA-DRA, TAP1, TAP2, LMP2, LMP7, RFX5, RFXAP, RFXANK, CIITA) and immune checkpoint genes such as PD1, LAG3, TIM3, CTLA4, PPP2CA, PPP2CB, PTPN6, PTPN22, PDCD1, HAVCR2, BTLA, CD160, TIGIT, CD96, CRTAM, TNFRSF10B, TNFRSF10A, CASP8, CASP10, CASP3, CASP6, CASP7, FADD, FAS, TGFBRII, TGFRBRI, SMAD2, SMAD3, SMAD4, SMAD10, SKI, SKIL, TGIF1, IL10RA, IL10RB, HMOX2, IL6R, IL6ST, EIF2AK4, CSK, PAG1, SIT, FOXP3, PRDM1, BATF, GUCY1A2, GUCY1A3, GUCY1 B2 and GUCY1B3. Preferably, the engineered immune cell further comprises suppressed or silenced expression of at least one gene selected from the group consisting of: TRAC, TRBC, HLA-A, HLA-B, HLA-C, B2M, RFX5, RFXAP, RFXANK, CIITA, PD1, LAG3, TIM3, CTLA4.

In an embodiment, the engineered immune cell is selected from the group consisting of a T cell, a macrophage, a dendritic cell, a monocyte, a NK cell or a NKT cell. Preferably, the T cell is a CD4+/CD8+ T cell, a CD4+ helper T cell, a CD8+ T cell, a tumor infiltrating cell, a memory T cell, a naive T cell, a γδ-T cells or an αβ-T cell. In an embodiment, the immune cell is derived from a stem cell, such as an adult stem cell, an embryonic stem cell, a cord blood stem cell, a progenitor cell, a bone marrow stem cell, an induced pluripotent stem cell, a totipotent stem cell, or a hematopoietic stem cell, among others.

In an embodiment, the present disclosure further provides a pharmaceutical composition comprises the engineered immune cell, the nucleic acid molecule, or the vector of the present disclosure, and one or more pharmaceutically acceptable excipients.

In a third aspect, the present disclosure further provides a method for treating a subject suffering from cancer, infection or autoimmune disease, comprising administering to the subject an effective amount of the immune cell or pharmaceutical composition of the present disclosure.

In an embodiment, the present disclosure also provides the use of the novel chimeric antigen receptor, the nucleic acid molecule, the vector, the engineered immune cell or the pharmaceutical composition according to the present disclosure in the preparation of a medicament for treating cancers, infections or autoimmune diseases.

The chimeric antigen receptor of the present disclosure has the following advantages. (1) It provides the intracellular region of the NK activating receptor or ligand thereof as an additional co-stimulatory domain, which has a stronger activation ability and improves killing ability of CAR cells, compared with the traditional co-stimulatory domains such as CD28 or 4-1BB alone. (2) In the case of general-purpose CAR cells, in order to inhibit the killing of exogenous CAR cells by host NK cells, it may be necessary to inhibit or silence the expression of endogenous NK activating receptor or ligand thereof in CAR cells. In this case, comprising the intracellular region of the corresponding inhibited or silenced NK activating receptor or ligand thereof in the CAR as a co-stimulatory domain can significantly improve the killing activity of CAR cells.

### Detailed Description of Embodiments

Unless otherwise indicated, all scientific and technical terms used herein have the same meaning as commonly understood by one of ordinary skill in the art to which the present disclosure belongs.

### Chimeric antigen receptor

As used herein, the term "chimeric antigen receptor" or "CAR" refers to an artificially constructed hybrid polypeptide that generally comprises a ligand-binding domain (such as an antibody or an antigen-binding portion thereof), a transmembrane domain, a co-stimulatory domain and an intracellular signaling domain which are connected by linkers. CARs are able to redirect the specificity and reactivity of T cells and other immune cells to selected targets in a non-MHC-restricted manner by means of the antigen-binding properties of antibodies. Non-MHC-restricted antigen recognition confers on CAR-expressing immune cells the ability to recognize antigens independently from antigen processing, thus bypassing major mechanisms of tumor escape.

In a first aspect, the present disclosure provides a chimeric antigen receptor comprising a ligand binding domain, a transmembrane domain, a co-stimulatory domain and an intracellular signaling domain, wherein the co-stimulatory domain comprises an intracellular region of an NK activating receptor or a ligand thereof.

A co-stimulatory domain is derived from an intracellular functional signaling domain from a costimulatory molecule, which comprises the entire intracellular region of the costimulatory molecule, or a functional fragment thereof. A "costimulatory molecule" refers to a cognate binding partner that specifically binds to a costimulatory ligand on a T cell, thereby mediating a costimulatory response (e.g., proliferation) of the T cell. Traditional chimeric antigen receptors use the intracellular region of CD28 or 4-1BB as the co-stimulatory domain. The chimeric antigen receptor of the present disclosure comprises a novel co-stimulatory domain, i.e., the intracellular region of an NK activating receptor or a ligand thereof. The present disclosure found that the addition of the intracellular region of an NK activating receptor or a ligand thereof as a novel co-stimulatory domain can significantly increase the killing activity of CAR cells on target cells, especially in the case that the expression of the corresponding endogenous NK activating receptor or ligand thereof in CAR cells is inhibited or silenced.

As used herein, the term "NK activating receptor" refers to a NK cell surface receptor that usually has an immunoreceptor tyrosine-based activation motif (ITAM) or is capable of activating NK cell activity. A "ligand of an NK activating receptor" or "NK activating ligand" refers to a molecule that binds to an NK activating receptor. The main function of NK activating receptor is to activate NK cells, causing them to exert cytotoxicity and release cytokines. NK cells regulate their activation state through a balance between activating receptors and inhibiting receptors. Under normal instance, NK inhibiting receptors play a dominant role in the signal transduction balance, inhibiting the activity of NK cells, thereby avoiding the killing of their own cells. However, when the expression of MHC-I molecules is abnormal, for example, when it is artificially inhibited or knocked out, NK activating receptors will take the lead, and NK cells will recognize such cells with abnormal MHC-I expression as "non-self" to kill.

In an embodiment, the NK activating receptor is 2B4. 2B4, also known as CD244, is a membrane protein widely expressed on the surface of NK cells, CD8+ T cells, monocytes and granulocytes. The extracellular region of 2B4 comprises a V-type immunoglobulin domain and a C2-type immunoglobulin-like domain, its transmembrane region does not contain any charged amino acids, and the intracellular region contains an immunoreceptor tyrosine-based inhibitory switch motif (ITSM), which can be recognized by the cytoplasmic SH2 region of adapter proteins SAP, EAT-2, DRT, etc. Cross-linking of 2B4 phosphorylates tyrosine in ITSM and recruits adapter proteins, and the complex formed by 2B4 and SAP can activate NK cells. 2B4 is not an independent receptor, but as a coactivator receptor of NK cells, and its initiation depends on the cooperation with other NCR receptors. The ligand of 2B4 is CD48, which is highly expressed on hematopoietic cell lines and some B lymphocytes. Thus, in an embodiment, the ligand of the NK activating receptor is CD48.

In an embodiment, the NK activating receptor is DNAM-1. DNAM-1, also known as CD226, is a main coactivating receptor that initiates NK cell function. DNAM-1 comprises an extracellular region of two immunoglobulin V-like domains, a transmembrane region, and a cytoplasmic region containing potential phosphorylation sites for tyrosine and serine residues. DNAM-1 is expressed on a variety of blood cells, comprising T cells, NK cells, NKT cells, monocytes, granulocytes and platelets. DNAM-1 is a common receptor for CD155 and CD112. CD112 is a herpes simplex virus receptor, is Ca2+-independent IgSF adhesion molecule , and belongs to the human nectin family, family members of which interact through homologous or heterologous forms to cause cell-cell adhesion. CD155 is similar in structure to nectin, and is also known Polyclonal Antibody to Poliovirus Receptor (PVR). Thus, in an embodiment, the ligand for the NK activating receptor is selected from CD112 and CD155.

In an embodiment, the NK activating receptor is LFA-1. LFA-1 is composed of two polypeptide chains linked by non-covalent bonds: the α subunit (CD11a) and the β subunit (CD18). The ligands of LFA-1 comprise members of the immunoglobulin superfamily ICAM1, ICAM2, and ICAM3, which are intercellular adhesion molecules and play an important role in positioning leukocytes to adhere to epithelial cells at the site of injury. ICAM1 is expressed on a variety of cells, such as lymphocytes, endothelial cells, monocytes, and tumor cells, and is regulated by the induction of cytokines. ICAM2 is expressed on leukocytes, endothelial cells and platelets, while ICAM3 is only expressed on leukocytes, and both are not regulated by the induction of cytokines. These three ligands bind to different regions within the α subunit of LFA-1, respectively. The interaction between ICAM molecules and LFA-1 provides the required costimulatory signals for cytotoxic T cells and NK cell-mediated immune killing, thereby activating the immune response. Studies have shown that ICAM1 can enhance the killing effect of NK cells mediated by NKG2D on colorectal cancer cells. Thus, in an embodiment, the ligand for the NK activating receptor is selected from ICAM1, ICAM2 and ICAM3.

In an embodiment, an intracellular region of a protein selected from the group consisting of 2B4, CD48, DNAM-1, CD112, CD155, LFA-1, ICAM1, ICAM2 or ICAM3, more preferably 2B4, CD155 and ICAM3 is used as a co-stimulatory domain of the chimeric antigen receptors of the present disclosure.

In an embodiment, the chimeric antigen receptor of the present disclosure comprises a 2B4 intracellular region as a co-stimulatory domain, which has at least 70%, preferably at least 80%, more preferably at least 90%, 95%, 97% or 99% or 100% sequence identity to the amino acid sequence represented by SEQ ID NO: 31, or its coding sequence has at least 70%, preferably at least 80%, more preferably at least 90% 95%, 97% or 99% or 100% sequence identity to the nucleotide sequence represented by SEQ ID NO: 32.

In an embodiment, the chimeric antigen receptor of the present disclosure comprises a CD155 intracellular region as a co-stimulatory domain, which has at least 70%, preferably at least 80%, more preferably at least 90%, 95%, 97% or 99% or 100% sequence identity to the amino acid sequence represented by SEQ ID NO: 29, or its coding sequence has at least 70%, preferably at least 80%, more preferably at least 90% 95%, 97% or 99% or 100% sequence identity to the nucleotide sequence represented by SEQ ID NO: 30.

In an embodiment, the chimeric antigen receptor of the present disclosure comprises a ICAM3 intracellular region as a co-stimulatory domain, which has at least 70%, preferably at least 80%, more preferably at least 90%, 95%, 97% or 99% or 100% sequence identity to the amino acid sequence represented by SEQ ID NO: 27, or its coding sequence has at least 70%, preferably at least 80%, more preferably at least 90% 95%, 97% or 99% or 100% sequence identity to the nucleotide sequence represented by SEQ ID NO: 28.

In an embodiment, the chimeric antigen receptor of the present disclosure further comprises intracellular regions of other known NK activating receptors or ligands thereof as co-stimulatory domains, examples of which include but are not limited to: NKG2 family proteins such as NKG2C, NKG2E, NKG2D, NKG2F, NKG2H and ligands that bind to them, such as HLA-E, Qa1b, MICA, MICB, ULBP1, ULBP2, ULBP3, ULBP4, ULBP5, ULBP6, Rae-1, H60, MULT1, etc.; natural cytotoxicity receptors (NCR) family, such as NKp30, NKp44, NKp46 or NKp80 and ligands that bind to them, such as B7-H6, BAG6, PfEMP1, HSPGS, AICL; KIR-S family, such as KIR2DS1, KIR2DS2, KIR2DS3, KIR2DS4, KIR2DS5, KIR3DS1, etc., and ligands that bind to them; coactivator receptors, such as CD2, and ligands that bind to them, such as CD58 and CD59; molecules related to IFNγ signaling pathways, such as STAT1, JAK1, IFNGR2, JAK2, IFNGR1, etc.

In addition to the intracellular region of the NK activating receptor or ligand thereof provided by the present disclosure as a co-stimulatory domain, the chimeric antigen receptor of the present disclosure may also comprise one or more additional co-stimulatory domains selected from the intracellular regions of the following proteins: TLR1, TLR2, TLR3, TLR4, TLR5, TLR6, TLR7, TLR8, TLR9, TLR10, CARD11, CD2, CD7, CD8, CD27, CD28, CD30, CD40, CD83, CD134 (OX40), CD137 (4-1BB), CD270 (HVEM), CD272 (BTLA), CD276 (B7-H3), CD278 (ICOS), CD357 (GITR), DAP10, DAP12, LAT, NKG2C, SLP76, PD-1, LIGHT, TRIM, CD94, LTB or ZAP70, preferably selected from 4-1BB, CD28, CD27, OX40 or a combination thereof, more preferably selected from 4-1BB, CD28. In an embodiment, the CAR of the present disclosure comprises the 2B4 intracellular region and the 4-1BB intracellular region as co-stimulatory domains. In an embodiment, the CAR of the present disclosure comprises CD155 intracellular region and 4-1BB intracellular region as co-stimulatory domains. In an embodiment, the CAR of the present disclosure comprises ICAM3 intracellular region and 4-1BB intracellular region as co-stimulatory domains. In an embodiment, the CAR of the present disclosure comprises the intracellular region of 2B4 and the intracellular region of CD28 as co-stimulatory domains. In an embodiment, the CAR of the present disclosure comprises CD155 intracellular region and CD28 intracellular region as co-stimulatory domains. In an embodiment, the CAR of the present disclosure comprises ICAM3 intracellular region and CD28 intracellular region as co-stimulatory domains.

The 4-1BB and CD28 intracellular regions as co-stimulatory domains are known to those skilled in the art. For example, the 4-1BB intracellular region has at least 70%, preferably at least 80%, more preferably at least 90%, 95%, 97% or 99% or 100% sequence identity to the amino acid sequence represented by SEQ ID NO: 9, or its coding sequence has at least 70%, preferably at least 80%, more preferably at least 90% 95%, 97% or 99% or 100% sequence identity to the nucleotide sequence represented by SEQ ID NO: 10. The CD28 intracellular region has at least 70%, preferably at least 80%, more preferably at least 90%, 95%, 97% or 99% or 100% sequence identity to the amino acid sequence represented by SEQ ID NO: 7, or its coding sequence has at least 70%, preferably at least 80%, more preferably at least 90% 95%, 97% or 99% or 100% sequence identity to the nucleotide sequence represented by SEQ ID NO: 8.

As used herein, "ligand binding domain" refers to any structure or functional variant thereof that can bind to a ligand (e.g. antigen). The ligand binding domain may be an antibody structure, including, but not limited to, monoclonal antibody, polyclonal antibody, recombinant antibody, human antibody, humanized antibody, murine antibody, chimeric antibody, and antigen-binding fragment thereof. For example, the ligand binding domain comprises, but is not limited to, an immunoglobulin molecule, Fab, Fab', F(ab')2, Fv fragment, scFv, disulfide-linked Fv (sdFv), antibody heavy chain variable region (VH) or light chain variable region (VL), linear antibody, heavy chain antibody, single domain antibody (sdAb), nanobody (Nb), recombinant fibronectin domain, anticalin and DARPIN, and so on, preferably selected from immunoglobulin molecule, Fab, Fab', F(ab')2, Fv fragment, scFv, linear antibody, heavy chain antibody, sdAb and nanobody, more preferably selected from Fab, Fab', Fab (ab')2, scFv, heavy chain antibody, sdAb and nanobody. In the present disclosure, the ligand binding domain may be monovalent or bivalent, and may be a monospecific, bispecific or multispecific antibody. "Fab" refers to any one of two identical fragments produced after an immunoglobulin molecule is cleaved by papain, and consists of an intact light chain and a heavy chain N-terminal part linked by a disulfide bond, wherein the heavy chain N-terminal part comprises a heavy chain variable region and CH1. Compared with intact IgG, Fab has no Fc fragment, has relatively high fluidity and tissue penetration ability, and can univalently bind to an antigen without mediating antibody effects.

A "single-chain antibody" or "scFv" is an antibody in which the heavy chain variable region (VH) and light chain variable region (VL) are linked by a linker. The optimal length and/or amino acid composition of the linker can be selected. The length of the linker can significantly affect the variable domain folding and interaction of scFv. In fact, if shorter linkers (e.g., between 5-10 amino acids) are used, intrachain folding can be prevented. For selection of linker size and composition, see, e.g., Hollinger et al., 1993 Proc Natl Acad. Sci. U.S.A. 90:6444-6448; U.S. Patent Application Publication Nos. 2005/0100543, 2005/0175606, 2007/0014794 and PCT Publication Nos. WO2006/020258 and WO2007/024715, the entire contents of which are incorporated herein by reference. A scFv may comprise VH and VL linked in any order, eg VH-linker-VL or VL-linker-VH.

"Heavy chain antibody" refers to an antibody naturally deficient in light chains, comprising a heavy chain variable region and normal CH2 and CH3 regions, found mainly in camelids. In recent years, a heavy chain antibody-like antigen receptor (new or nurse shark antigen receptor, NAR) without light chain or accompanying other protein molecules was found in cartilaginous fish. Because NAR molecules are similar to Ig subtypes in terms of transmembrane and secretion, they are also called immunoglobulin new antigen receptors (Ig new antigen receptors, IgNAR).

"Single domain antibody" or "sdAb" refers to a genetically engineered antibody consisting only of the light chain antibody variable region or the heavy chain antibody variable region. The sdAb found in camelids only contains the heavy chain antibody variable region, also known as VHH or "nanobody", which has comparable structural stability and antigen-binding activity to the original heavy chain antibody, and is currently the smallest unit known to bind the target antigen. A basic VHH has the following structure from N-terminus to C-terminus: FR1-CDR1-FR2-CDR2-FR3-CDR3-FR4, where CDR refers to complementarity determining regions and FR refers to framework regions.

The term "functional variant" or "functional fragment" refers to a variant that substantially comprises the amino acid sequence of a parent, but, compared with the parent amino acid sequence, contains at least one amino acid modification (i.e., substitution, deletion, or insertion), provided that the variant retains the biological activity of the parent amino acid sequence. For example, for an antibody, a functional fragment thereof is the antigen-binding portion thereof. In an embodiment, the amino acid modification is preferably a conservative modification.

As used herein, the term "conservative modification" refers to amino acid modification that does not significantly affect or alter the binding characteristics of the antibody or antibody fragment containing the amino acid sequence. These conservative modifications comprise amino acid substitution, addition, and deletion. The modifications can be introduced into the chimeric antigen receptor of the present disclosure by standard techniques known in the art, such as site-directed mutagenesis and PCR-mediated mutagenesis. The conservative amino acid substitution is a substitution in which the amino acid residue is replaced with an amino acid residue having a similar side chain. Amino acid residue families having a similar side chain have been defined in the art, comprising basic side chain (e.g., lysine, arginine, histidine), acidic side chain (e.g., aspartic acid, glutamic acid), uncharged polar side chain (e.g., glycine, asparagine, glutamine, serine, threonine, tyrosine, cysteine), nonpolar side chain (e.g., alanine, valine, leucine, isoleucine, proline, phenylalanine, methionine, tryptophan), β-branched side chain (e.g., threonine, valine, isoleucine), and aromatic side chain (e.g., tyrosine, phenylalanine, tryptophan, histidine). The conservative modifications may be selected, for example, based on polarity, charge, solubility, hydrophobicity, hydrophilicity, and/or similarity in amphiphilic properties of residues involved.

Thus, the "functional variant" or "functional fragment" has at least 75%, preferably at least 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the parent amino acid sequence, and retains the biological activity, e.g., binding activity, of the parent amino acid.

As used herein, the term "sequence identity" indicates the degree to which two (nucleotide or amino acid) sequences have the same residue at the same position in an alignment, and is generally expressed by percentage. Preferably, the identity is determined over the entire length of the sequences being compared. Thus, two copies with completely identical sequences have 100% identity. Those skilled in the art will recognize that some algorithms can be used to determine sequence identity using standard parameters, for example, Blast (Altschul et al. (1997) Nucleic Acids Res. 25:3389-3402), Blast2 (Altschul et al. (1990) J. Mol. Biol. 215:403-410), Smith-Waterman (Smith et al. (1981) J. Mol. Biol. 147:195-197), and ClustalW.

The selection of ligand binding domain depends on the cell surface marker on a target cell to be recognized and associated with a specific disease state, for example, a tumor specific antigen or a tumor associated antigen. Thus, in an embodiment, the ligand binding domain of the present disclosure binds to one or more targets selected from the group consisting of: CD2, CD3, CD4, CD5, CD7, CD8, CD14, CD15, CD46, CD70, TSHR, CD19, CD123, CD22, CD30, CD171, CS-1, CLL-1, CD33, EGFRvIII, GD2, GD3, BCMA, Tn Ag, PSMA, ROR1, FLT3, FAP, TAG72, CD38, CD44v6, CEA, EPCAM, B7H3, KIT, IL-13Ra2, mesothelin, IL-1 1Ra, PSCA, PRSS21, VEGFR2, LewisY, CD24, PDGFR-β, SSEA-4, CD20, Folate receptor α, ERBB2 (Her2/neu), MUC1, EGFR, NCAM, Prostase, PAP, ELF2M, Ephrin B2, IGF-I receptor, CAlX, LMP2, gploo, bcr-abl, tyrosinase, EphA2, Fucosyl GM1, sLe, GM3, TGS5, HMWMAA, o-acetyl-GD2, Folate receptor β, TEM1/CD248, TEM7R, CLDN6, GPRC5D, CXORF61, CD97, CD 179a, ALK, polysialic acid, PLAC1, GloboH, NY-BR-1, UPK2, HAVCR1, ADRB3, PANX3, GPR20, LY6K, OR51E2, TARP, WT1, NY-ESO-1, LAGE-1a, MAGE-A1, legumain, HPV E6, E7, MAGE A1, ETV6-AML, sperm protein 17, XAGE1, Tie 2, MAD-CT-1, MAD-CT-2, Fos associated antigen 1, p53, p53 mutant, prostate specific protein, survivin and telomerase, PCTA-1/Galectin 8, MelanA/MART1, Ras mutant, hTERT, sarcoma translocation breakpoint, ML-IAP, ERG (TMPRSS2 ETS fusion gene), NA17, PAX3, androgen receptor, Cyclin B1, MYCN, RhoC, TRP-2, CYP1B 1, BORIS, SART3, PAX5, OY-TES 1, LCK, AKAP-4, SSX2, RAGE-1, human telomerase reverse transcriptase, RU1, RU2, intestinal tract carboxylesterase, mut hsp70-2, CD79a, CD79b, CD72, LAIR1, FCAR, LILRA2, CD300LF, CLEC12A, BST2, EMR2, LY75, GPC3, FCRL5, IGLL1, PD1, PDL1, PDL2, TGF β, APRIL, Claudin18.2, NKG2D, NKG2D ligand and/or pathogen-specific antigen, biotinylated molecule, molecule expressed by HIV, HCV, HBV, and/or other pathogens; and/or neo-epitope or neoantigen. Preferably, the target is selected from the group consisting of: CD7, CD19, CD20, CD22, BAFF-R, CD33, EGFRvIII, BCMA, GPRC5D, PSMA, ROR1, FAP, ERBB2 (Her2/neu), MUC1, EGFR, CAlX, WT1, NY-ESO-1, CD79a, CD79b, GPC3, Claudin18.2, NKG2D, and any combination thereof. Depending on the antigen to be targeted, the CAR of the present disclosure may be designed to comprise a ligand binding domain specific for the antigen. For example, if CD19 is the antigen to be targeted, a CD19 antibody can be used as a ligand binding domain of the present disclosure.

In an embodiment, the chimeric antigen receptor of the present disclosure targets CD19. Therefore, in a preferred embodiment, the chimeric antigen receptor of the present disclosure comprises an anti-CD19 antibody, which comprises: (i) CDR-L1, CDR-L2 and CDR-L3 as shown in SEQ ID NO: 33, 34 and 35, respectively, and CDR-H1, CDR-H2 and CDR-H3 as shown in SEQ ID NO: 36, 37 and 38, respectively; or (ii) CDR-L1, CDR-L2 and CDR-L3 as shown in SEQ ID NO: 39, 40 and 41, respectively, and CDR-H1, CDR-H2 and CDR-H3 as shown in SEQ ID NO: 42, 43 and 44, respectively. Preferably, the anti-CD19 antibody comprises a light chain variable region sequence having at least 70%, preferably at least 80%, more preferably at least 90%, 95%, 97%, 99% or 100% sequence identity to the amino acid sequence shown at positions 1-107 of SEQ ID NO: 1 or at positions 1-107 of SEQ ID NO: 25, and a heavy chain variable region sequence having at least 70%, preferably at least 80%, more preferably at least 90%, 95%, 97%, 99% or 100% sequence identity to the amino acid sequence shown at positions 123-242 of SEQ ID NO: 1 or at positions 123-238 of SEQ ID NO: 25.

As used herein, the term "transmembrane domain" refers to a polypeptide structure that enables expression of a chimeric antigen receptor on the surface of an immune cell (e.g., a lymphocyte, an NK cell, or an NKT cell), and guides a cellular response of the immune cell against the target cell. The transmembrane domain may be natural or synthetic, and also may be derived from any membrane-bound protein or transmembrane protein. The transmembrane domain is capable of signaling when the chimeric antigen receptor binds to the target antigen. The transmembrane domains particularly suitable for use in the present disclosure may be derived from, for example, a TCR α chain, a TCR β chain, a TCR γ chain, a TCR δ chain, a CD3 ζ subunit, a CD3 ε subunit, a CD3 γ subunit, a CD3 δ subunit, CD45, CD4, CD5, CD8 α, CD9, CD16, CD22, CD33, CD28, CD37, CD64, CD80, CD86, CD134, CD137, CD154, and functional fragments thereof. Alternatively, the transmembrane domain may be synthesized and may mainly contain a hydrophobic residue such as leucine and valine. Preferably, the transmembrane domain is derived from a CD8 α chain or CD28, which has at least 70%, preferably at least 80%, more preferably at least 90%, 95%, 97% or 99% or 100% sequence identity to an amino acid sequence represented by SEQ ID NO: 3 or 5, or an encoding sequence thereof has at least 70%, preferably at least 80%, more preferably at least 90%, 95%, 97% or 99% or 100% sequence identity to a nucleotide sequence represented by SEQ ID NO: 4 or 6.

In an embodiment, the chimeric antigen receptor of the present disclosure further may comprise a hinge region located between the ligand binding domain and the transmembrane domain. As used herein, the term "hinge region" generally refers to any oligopeptide or polypeptide that functions to link a transmembrane domain to a ligand binding domain. Specifically, the hinge region serves to provide greater flexibility and accessibility to the ligand binding domain. The hinge region may contain up to 300 amino acids, preferably 10 to 100 amino acids and most preferably 25 to 50 amino acids. The hinge region may be completely or partially derived from a natural molecule, for example, completely or partially from the extracellular region of CD8, CD4 or CD28, or completely or partially from an antibody constant region. Alternatively, the hinge region may be a synthetic sequence corresponding to a naturally occurring hinge sequence, or may be a completely synthetic hinge sequence. In a preferred embodiment, the hinge region contains a hinge region portion of a CD8 α chain, an Fc γ RIII α receptor, CD28, an IgG4, or an IgG1, more preferably a hinge from CD8α, CD28 or IgG4, which has at least 70%, preferably at least 80%, more preferably at least 90%, 95%, 97% or 99% or 100% sequence identity to an amino acid sequence represented by SEQ ID NO: 19, 21 or 23, or an encoding sequence thereof has at least 70%, preferably at least 80%, more preferably at least 90%, 95%, 97% or 99% or 100% sequence identity to a nucleotide sequence represented by SEQ ID NO: 20, 22 or 24.

As used herein, the term "intracellular signaling domain" refers to a protein portion that transduces an effector function signal and guides a cell to perform a specified function, also referred to as a primary signaling domain. The intracellular signaling domain is responsible for intracellular primary signaling after the ligand binding domain binds to the antigen, thus causing activation of immune cell and immune reaction. In other words, the intracellular signaling domain is responsible for activating at least one of the normal effector functions of the immune cells in which the CAR is expressed. For example, the effector functions of T cell can be cytolytic activity or auxiliary activity, comprising secretion of cytokines.

In an embodiment, the intracellular signaling domain of the chimeric antigen receptor of the present disclosure may be cytoplasmic sequences of a T cell receptor and a co-receptor, upon antigen receptor binding, which act together to initiate primary signaling, as well as any derivative or variant of these sequences and any synthetic sequence having the same or similar function. The intracellular signaling domain may contain many immunoreceptor tyrosine-based activation motifs (ITAM). Non-limiting examples of intracellular signaling domain of the present disclosure include, but are not limited to, intracellular regions of FcR γ, FcR β, CD3 γ, CD3 δ, CD3 ε, CD3 ζ, CD22, CD79a, CD79b, and CD66d. In a preferred embodiment, the signaling domain of the CAR of the present disclosure may contain a CD3 ζ intracellular region, which has at least 70%, preferably at least 80%, more preferably at least 90%, 95%, 97%, or 99% or 100% sequence identity to an amino acid sequence represented by SEQ ID NO: 11 or 13, or an encoding sequence thereof has at least 70%, preferably at least 80%, more preferably at least 90%, 95%, 97%, or 99% or 100% sequence identity to a nucleotide sequence represented by SEQ ID NO: 12 or 14.

In an embodiment, the co-stimulatory domain and the intracellular signaling domain may be operably linked in any order. For example, the co-stimulatory domain may be located membrane proximally and the intracellular signaling domain membrane distally or the co-stimulatory domain may be located membrane distally and the intracellular signaling domain membrane proximally. When two or more co-stimulatory domains are contained, the co-stimulatory domains may be located on one or both sides of the intracellular signaling domain.

In an embodiment, the CAR of the present disclosure further may comprise a signal peptide such that when it is expressed in a cell such as a T cell, the nascent protein is directed to the endoplasmic reticulum and subsequently to the cell surface. The core of the signal peptide may contain a long hydrophobic amino acid segment, which has a tendency to form a single α-helix. At the end of the signal peptide, there is usually an amino acid segment recognized and cleaved by signal peptidase. The signal peptidase can cleave during or after translocation, so as to generate free signal peptide and mature protein. Then, the free signal peptide is digested by a specific protease. Signal peptides that can be used in the present disclosure are well known to those skilled in the art, for example, signal peptides derived from CD8 α, IgG1, GM-CSFRα, B2M, and so on. In an embodiment, the signal peptide that can be used in the present disclosure has at least 70%, preferably at least 80%, more preferably at least 90%, 95%, 97%, or 99% or 100% sequence identity to an amino acid sequence represented by SEQ ID NO: 15 or 17, or an encoding sequence of the signal peptide has at least 70%, preferably at least 80%, more preferably at least 90%, 95%, 97%, or 99% or 100% sequence identity to a nucleotide sequence represented by SEQ ID NO: 16 or 18.

In an embodiment, the CAR of the present disclosure further may comprise a switch structure to regulate the expression time of the CAR. For example, the switch structure may be in a form of dimerization domain, which causes a conformational change by binding to a corresponding ligand thereof, and exposes the extracellular binding domain to enable its binding to a targeted antigen, thereby activating a signaling pathway. Alternatively, a switch domain also may be used to link the binding domain and signaling domain, respectively, and only when the switch domains are bound to each other (for example, in the presence of an inducing compound), the binding domain and the signaling domain can be linked together through a dimer, thereby activating signaling pathway. The switch structure also can be in the form of a masking peptide. The masking peptide can shield the extracellular binding domain, and prevent it from binding to the targeted antigen. When the masking peptide is cleaved by, for example, a protease, the extracellular binding domain is exposed, making it become a "normal" CAR structure. A variety of switch structures known to those skilled in the art can be used in the present disclosure.

In an embodiment, the CAR of the present disclosure further may comprise a suicide gene, to make it express a cell death signal that can be induced by an exogenous substance, so as to eliminate the CAR cell when needed (e.g., when serious toxic side effects are produced). For example, the suicide gene may be in the form of an inserted epitope, e.g., a CD20 epitope, an RQR8, etc., and when needed, the CAR cell can be eliminated by adding an antibody or reagent that targets these epitopes. The suicide gene also may be herpes simplex virus thymidine kinase (HSV-TK), which gene can induce the cell to die when receiving ganciclovir treatment. The suicide gene further may be iCaspase-9, and dimerization of iCaspase-9 can be induced by a chemical induction drug such as AP1903 and AP20187, so as to activate the downstream Caspase3 molecule, and cause apoptosis. A variety of suicide genes known to those of skill in the art can be used in the present disclosure.

### Nucleic Acid and Vector

The present disclosure also provides a nucleic acid molecule comprising a nucleic acid sequence encoding the chimeric antigen receptor of the present disclosure. The present disclosure also provides a vector comprising such a nucleic acid molecule.

As used herein, the term "nucleic acid molecule" comprises a sequence of ribonucleotide and deoxyribonucleotide, such as modified or unmodified RNA or DNA, each in single-stranded and/or double-stranded form, linear or circular, or their mixtures (comprising hybrid molecules). Thus, the nucleic acid according to the present disclosure comprises DNA (e.g. dsDNA, ssDNA, cDNA), RNA (e.g. dsRNA, ssRNA, mRNA, ivtRNA), their combinations or derivatives (e.g. PNA). Preferably, the nucleic acid is DNA or RNA, more preferably mRNA.

The nucleic acid may contain a conventional phosphodiester bond or an unconventional bond (e.g., amide bond, such as found in peptide nucleic acid (PNA)). The nucleic acid of the present disclosure further may contain one or more modified bases, such as, for example, trityl base and uncommon base (such as inosine). Other modifications also can be contemplated, comprising chemical, enzymatic, or metabolic modifications, so long as the multi-chain CAR of the present disclosure can be expressed from polynucleotides. The nucleic acid can be provided in isolated form. In an embodiment, the nucleic acid also may comprise a regulatory sequence, such as a transcriptional control element (comprising a promoter, an enhancer, an operon, a repressor, and a transcription termination signal), ribosome binding sites, and introns.

The nucleic acid sequences of the present disclosure can be codon-optimized for optimal expression in a desired host cell (e.g., immune cell); or for expression in a bacterial, yeast, or insect cell. Codon optimization refers to substitution of a codon in the target sequence that is generally rare in highly expressed genes of a given species with a codon that is generally common in highly expressed genes of such species, and the codons before and after the substitution encode the same amino acid. Therefore, the selection of an optimal codon depends on the codon usage preference of the host genome.

As used herein, the term "vector" is an intermediary nucleic acid molecule used to transfer (exogenous) genetic material into a host cell, and in the host cell the nucleic acid molecule can be, for example, replicated and/or expressed.

The vector generally comprises targeting vectors and expression vectors. The "targeting vector" is a medium that delivers an isolated nucleic acid to the interior of a cell by, for example, homologous recombination or by using a hybrid recombinase of a sequence at specific target site. The "expression vector" is a vector used for transcription of heterologous nucleic acid sequences (for example, those sequences encoding the chimeric antigen receptor polypeptides of the present disclosure) in suitable host cells and the translation of their mRNAs. Suitable vectors that can be used in the present disclosure are known in the art, and many are commercially available. In an embodiment, the vector of the present disclosure comprises, but is not limited to, plasmid, virus (e.g., retrovirus, lentivirus, adenovirus, vaccinia virus, Rous sarcoma virus (RSV), polyoma virus, and adeno-associated virus (AAV), etc.), bacteriophage, phagemid, cosmid, and artificial chromosome (comprising BAC and YAC). The vector itself is usually a nucleotide sequence, and usually is a DNA sequence containing an insert (transgene) and a larger sequence as "backbone" of the vector. Engineered vector typically also contains an origin autonomously replicating in the host cell (if stable expression of polynucleotide is desired), a selectable marker, and a restriction enzyme cleavage site (e.g., a multiple cloning site, MCS). The vectors may additionally contain elements such as a promoter, a poly-A tail (polyA), 3' UTR, an enhancer, a terminator, an insulator, an operon, a selectable marker, a reporter gene, a targeting sequence, and/or a protein purification tag. In a specific embodiment, the vector is an *in vitro* transcription vector.

### Engineered immune cell

The present disclosure provides an engineered immune cell comprising the chimeric antigen receptor, the nucleic acid molecule or the vector of the present disclosure.

As used herein, the term "immune cell" refers to any cell of the immune system that has one or more effector functions (e.g., cytotoxic cell killing activity, secretion of cytokines, induction of ADCC and/or CDC). For example, the immune cell may be a T cell, a macrophage, a dendritic cell, a monocyte, an NK cell, and/or an NKT cell. In an embodiment, the immune cell is derived from a stem cell, such as an adult stem cell, an embryonic stem cell, a cord blood stem cell, a progenitor cell, a bone marrow stem cell, an induced pluripotent stem cell, a totipotent stem cell, or a hematopoietic stem cell, and so on. Preferably, the immune cell is a T cell. The T cell may be any T cell, such as *in vitro* cultured T cell, for example, primary T cell, or T cell from *in vitro* cultured T cell line, e.g., Jurkat, SupT1, etc., or T cell obtained from a subject. Examples of subject comprise humans, dogs, cats, mice, rats, and transgenic species thereof. The T cell can be obtained from a variety of sources, comprising peripheral blood monocytes, bone marrow, lymph node tissue, umbilical blood, thymus tissue, tissue from sites of infection, ascites, pleural effusion, spleen tissue, and tumors. The T cell also may be concentrated or purified. The T cell may be at any stage of development comprising, but not limited to, a CD4+/CD8+ T cell, a CD4+ helper T cell (e.g., Th1 and Th2 cells), CD8+ T cell (e.g., cytotoxic T cell), CD4-CD8- T cell, tumor infiltrating cell, memory T cell, naive T cell, γδ-T cell, αβ-T cell, etc. In a preferred embodiment, the immune cell is a human T cell. The T cell can be isolated from the blood of a subject using a variety of techniques known to those of skill in the art, such as Ficoll.

The nucleic acid sequence encoding the chimeric antigen receptor and optional exogenous gene can be introduced into an immune cell using conventional methods known in the art (e.g., by transduction, transfection, transformation). "Transfection" is a process of introducing a nucleic acid molecule or polynucleotide (comprising a vector) into a target cell. An example is RNA transfection, i.e., the process of introducing RNA (such as *in vitro* transcribed RNA, ivtRNA) into a host cell. This term is mainly used for a non-viral method in eukaryotic cells. The term "transduction" is generally used to describe virus-mediated transfer of nucleic acid molecules or polynucleotides. Transfection of animal cells typically involves opening transient pores or "holes" in the cell membrane, so as to allow uptake of material. Transfection may be carried out using calcium phosphate, by electroporation, by extrusion of cells, or by mixing cationic lipids with the material so as to produce liposomes which fuse with the cell membrane and deposit their cargo into the interior. Exemplary techniques for transfecting eukaryotic host cells comprise lipid vesicle-mediated uptake, heat shock-mediated uptake, calcium phosphate-mediated transfection (calcium phosphate/DNA co-precipitation), microinjection, and electroporation. The term "transformation" is used to describe the non-virus transfer of a nucleic acid molecule or polynucleotide (comprising a vector) to bacteria, and also to non-animal eukaryotic cells (comprising plant cells). Thus, the transformation is a genetic alteration of bacterial or non-animal eukaryotic cells, which is produced by direct uptake of a cell membrane from its surroundings and subsequent incorporation of exogenous genetic material (nucleic acid molecule). The transformation can be achieved by artificial means. In order for transformation to occur, the cell or bacterium must be in a competent state. For prokaryotic transformation, the techniques may comprise heat shock-mediated uptake, fusion to bacterial protoplasts of intact cells, microinjection, and electroporation. After the nucleic acid or vector is introduced into the immune cells, those skilled in the art can amplify and activate the obtained immune cells by conventional techniques.

In an embodiment, the expression of the corresponding endogenous NK activating receptor or ligand serving as a co-stimulatory domain is inhibited or silenced in the engineered immune cell. When preparing a general-purpose CAR cell to reduce the risk of graft-versus-host disease, endogenous MHC-related genes in CAR cells, such as HLA, B2M, CIITA, RFX5, etc., are usually knocked out to prevent CAR cells from being attacked by T cells having receptors and thereby affecting the survival and development of CAR cells. However, certain NK inhibitory receptors, such as NKG2A, also bind MHC-associated genes. Such binding allows the normally inhibitory signals in NK cells to take over, recognizing the cell as "self" and not attacking. Therefore, when the expression of endogenous MHC-related genes in CAR cells is inhibited or silenced, the inhibitory signal of NK cells in the patient will be eliminated, and the activation signal will dominate. As a result, the CAR cells are recognized as "non-self" by the NK cells in the patient's body for killing. In this case, it may be necessary to inhibit or silence the expression of endogenous NK activating receptors or ligands thereof in CAR cells to reduce the activating signals of NK cells and reduce the killing of exogenous CAR cells by NK cells in vivo.

The inventors have found for the first time that in CAR cells in which the expression of NK activating receptors or ligands thereof is inhibited or silenced, comprising the intracellular region of the corresponding inhibited or silenced NK activating receptor or ligand thereof as a co-stimulatory domain can significantly improve the killing activity of CAR cells; in CAR cells in which the expression of NK activating ligands is inhibited or silenced, comprising the intracellular region of the corresponding inhibited or silenced NK activating ligand or receptor of as a co-stimulatory domain can significantly improve the killing activity of CAR cells.

Therefore, in an embodiment, the expression of the endogenous NK activating ligand CD155 of the engineered immune cells of the present disclosure is inhibited or silenced, and the expressed chimeric antigen receptor comprises the intracellular region of CD155 or the intracellular region of a receptor for CD155 (e.g., DNAM-1) as a co-stimulatory domain. In an embodiment, the expression of the endogenous NK activating ligand ICAM3 of the engineered immune cells of the present disclosure is inhibited or silenced, and the expressed chimeric antigen receptor comprises the intracellular region of ICAM3 or the intracellular region of a receptor for ICAM3 (e.g., LFA-1) as a co-stimulatory domain. In an embodiment, the expression of the endogenous NK activating receptor 2B4 of the engineered immune cells of the present disclosure is inhibited or silenced, and the expressed chimeric antigen receptor comprises the intracellular region of 2B4 or the intracellular region of a ligand for 2B4 (e.g., CD48) as a co-stimulatory domain.

In addition, the inventors also unexpectedly found that individually inhibiting or silencing the expression of NK activating ligands or receptors thereof can increase the proliferation level and prolong the persistence of engineered immune cells (such as CAR-T cells) in vivo, thereby improving the sustained killing effect on tumor cells and improving the tumor suppression effect in vivo.

Therefore, in an embodiment, the present disclosure also provides an engineered immune cell, in which the expression of a NK activating receptor or a ligand thereof is inhibited or silenced, wherein the NK activating receptor is selected from 2B4, DNAM-1 and LFA-1, and the ligand of the NK activating receptor is selected from CD48, CD112, CD155, ICAM1, ICAM2 and ICAM3, preferably the expression of CD48 or ICAM3 is inhibited or silenced.

In a preferred embodiment, the engineered immune cell in which the expression of an NK activating ligand or receptor thereof is inhibited or silenced also expresses a chimeric antigen receptor or a recombinant T cell receptor. More preferably, the engineered immune cell in which the expression of NK activating ligand or receptor thereof is suppressed or silenced also expresses a chimeric antigen receptor comprising a ligand binding domain, a transmembrane domain, a co-stimulatory domain and an intracellular signaling domain, wherein the co-stimulatory domain is selected from the signaling domain of CD27, CD28, CD134, CD137 or CD278 or a combination thereof. In another embodiment, the chimeric antigen receptor expressed by the engineered immune cell in which the expression of an NK activating ligand or receptor thereof is inhibited or silenced does not comprise the intracellular region of the NK activating ligand or receptor thereof.

In an embodiment, in order to reduce the risk of graft-versus-host disease, the engineered immune cell further comprises suppressed or silenced expression of at least one gene selected from the group consisting of: CD52, GR, dCK, TCR/CD3 genes (e.g. TRAC, TRBC, CD3γ, CD3δ, CD3ε, CD3ζ), MHC related genes (HLA-A, HLA-B, HLA-C, B2M, HLA-DPA, HLA-DQ, HLA-DRA, TAP1, TAP2, LMP2, LMP7, RFX5, RFXAP, RFXANK, CIITA) and immune checkpoint genes such as PD1, LAG3, TIM3, CTLA4, PPP2CA, PPP2CB, PTPN6, PTPN22, PDCD1, HAVCR2, BTLA, CD160, TIGIT, CD96, CRTAM, TNFRSF10B, TNFRSF10A, CASP8, CASP10, CASP3, CASP6, CASP7, FADD, FAS, TGFBRII, TGFRBRI, SMAD2, SMAD3, SMAD4, SMAD10, SKI, SKIL, TGIF1, IL10RA, IL10RB, HMOX2, IL6R, IL6ST, EIF2AK4, CSK, PAG1, SIT, FOXP3, PRDM1, BATF, GUCY1A2, GUCY1A3, GUCY1B2 and GUCY1B3. Preferably, the engineered immune cell further comprises suppressed or silenced expression of at least one gene selected from the group consisting of: TRAC, TRBC, HLA-A, HLA-B, HLA-C, B2M, RFX5, RFXAP, RFXANK, CIITA, PD1, LAG3, TIM3, CTLA4, more preferably TRAC, TRBC, HLA-A, HLA-B, HLA-C, B2M, RFX5, RFXAP, RFXANK, CIITA.

Methods of inhibiting gene expression or silencing genes are well known to those skilled in the art. For example, antisense RNA, RNA decoys, RNA aptamers, siRNA, shRNA/miRNA, trans dominant negative protein (TNP), chimeric/fusion proteins, chemokine ligands, anti-infective cellular proteins, intracellular antibodies (sFv), nucleoside analogs (NRTI), non-nucleoside analogs (NNRTI), integrase inhibitors (oligonucleotides, dinucleotides, and chemical agents), and protease inhibitors may be used to inhibit the expression of genes. Alternatively, genes can also be silenced by DNA fragmentation mediated by for example meganucleases, zinc finger nucleases, TALE nucleases or Cas enzymes in CRISPR systems.

### Pharmaceutical composition

The present disclosure further provides a pharmaceutical composition, which comprises the chimeric antigen receptor, the nucleic acid molecule, the vector or the engineered immune cell described in the present disclosure as an active agent, and a variety of pharmaceutically acceptable excipients.

As used herein, the term "pharmaceutically acceptable excipient" refers to a vector and/or excipient that is pharmacologically and/or physiologically compatible (i.e., capable of triggering a desired therapeutic effect without causing any undesired local or systemic effects) with the subject and active ingredient, and it is well known in the art (see, e.g., Remington's Pharmaceutical Sciences. Edited by Gennaro AR, 19th ed. Pennsylvania: Mack Publishing Company, 1995). Examples of pharmaceutically acceptable excipient include, but are not limited to, filler, binder, disintegrant, coating agent, adsorbent, anti-adherent, glidant, antioxidant, flavoring agent, colorant, sweetener, solvent, co-solvent, buffer agent, chelating agent, surfactant, diluent, wetting agent, preservative, emulsifier, cladding agent, isotonic agent, absorption delaying agent, stabilizer, and tension regulator. It is known to those skilled in the art to select a suitable excipient to prepare the desired pharmaceutical composition of the present disclosure. Exemplary excipients for use in the pharmaceutical composition of the present disclosure comprise saline, buffered saline, dextrose, and water. Generally, the selection of a suitable excipient depends, in particular, on the active agent used, the disease to be treated, and the desired dosage form of the pharmaceutical composition.

The pharmaceutical composition according to the present disclosure is suitable for multiple routes of administration. Generally, the application is parenterally accomplished. Parenteral delivery methods comprise topical, intraarterial, intramuscular, subcutaneous, intramedullary, intrathecal, intraventricular, intravenous, intraperitoneal, intrauterine, intravaginal, sublingual, or intranasal administration.

The pharmaceutical composition according to the present disclosure also can be prepared in various forms, such as solid, liquid, gaseous or lyophilized forms, particularly the pharmaceutical composition can be prepared in the form of ointment, cream, transdermal patch, gel, powder, tablet, solution, aerosol, granule, pill, suspension, emulsion, capsule, syrup, elixir, extract, tincture or liquid extract, or in a form particularly suitable for the desired method of administration. Processes known in the present disclosure for producing a medicament may comprise, for example, conventional mixing, dissolving, granulating, dragee-making, grinding, emulsifying, encapsulating, embedding or lyophilizing process. The pharmaceutical composition comprising, for example, the immune cell as described herein is generally provided in a form of solution, and preferably comprises a pharmaceutically acceptable buffer agent.

The pharmaceutical composition according to the present disclosure further may be administered in combination with one or more other agents suitable for the treatment and/or prophylaxis of diseases to be treated. Preferred examples of agent suitable for the combination comprise known anti-cancer medicaments such as cisplatin, maytansine derivatives, rachelmycin, calicheamicin, docetaxel, etoposide, gemcitabine, ifosfamide, irinotecan, melphalan, mitoxantrone, sorfimer sodiumphotofrin II, temozolomide, topotecan, trimetreate glucuronate, auristatin E, vincristine and doxorubicin; peptide cytotoxins, such as ricin, diphtheria toxin, pseudomonas exotoxin A, DNase and RNase; radionuclides such as iodine 131, rhenium 186, indium 111, iridium 90, bismuth 210 and 213, actinides 225 and astatine 213; prodrugs such as antibody-directed enzyme prodrugs; immunostimulatory agents such as platelet factor 4, and melanoma growth stimulating protein; antibodies or fragments thereof, such as anti-CD3 antibodies or fragments thereof, complement activators, heterologous protein domains, homologous protein domains, viral/bacterial protein domains and viral/bacterial peptides. In addition, the pharmaceutical composition of the present disclosure also can be used in combination with one or more other treatment methods, such as chemotherapy and radiotherapy.

### Therapeutic use

The present disclosure further provides a method of treating a subject with cancer, infection or autoimmune disease, comprising administering to the subject an effective amount of the immune cell or the pharmaceutical composition according to the present disclosure. Therefore, the present disclosure also encompasses use of the chimeric antigen receptor, the nucleic acid molecule, the vector, the engineered immune cell and the pharmaceutical composition in the preparation of a medicament for treating cancer, infection, or autoimmune diseases.

In an embodiment, an effective amount of the immune cell and/or the pharmaceutical composition of the present disclosure is directly administered to the subject.

In another embodiment, the treatment method of the present disclosure is *ex vivo* treatment. Specifically, the method comprises the steps of: (a) providing a sample, the sample containing an immune cell; (b) introducing the chimeric antigen receptor of the present disclosure and an exogenous gene (if present) into the immune cell *in vitro* and optionally inhibiting or silencing the expression of a specific gene in the immune cell (if desired) to obtain a modified immune cell, and (c) administering the modified immune cell to the subject in need thereof. Preferably, the immune cell provided in step (a) is selected from a macrophage, a dendritic cell, a monocyte, a T cell, an NK cell, and/or an NKT cell; and the immune cell can be obtained from the sample (particularly a blood sample) of the subject by conventional methods known in the art. However, other immune cells capable of expressing the chimeric antigen receptor and exogenous gene of the present disclosure and exerting the desired biological effect function as described herein also can be used. Besides, the immune cells generally selected are compatible with the subject's immune system, i.e., it is preferred that the immune cells do not trigger an immunogenic response. For example, a "universal recipient cell", i.e., a universally compatible lymphocyte exerting a desired biological effect function and being capable of growing and amplifying *in vitro,* can be used. The use of such cells will not require obtaining and/or providing the subject's own lymphocyte. The *ex vivo* introduction of step (c) may be carried out by introducing the nucleic acid or vector described herein into the immune cell via electroporation or by infecting the immune cell with a viral vector, wherein the viral vector is a lentiviral vector, adenoviral vector, adeno-associated viral vector or retroviral vector as previously described. Other conceivable methods comprise using a transfection reagent (such as a liposome) or transient RNA transfection.

In an embodiment, the immune cell is an autologous or allogeneic cell, preferably T cell, macrophage, dendritic cell, monocyte, NK cell and/or NKT cell, more preferably T cell, NK cell or NKT cell.

As used herein, the term "autologous" means that any material derived from an individual will be later re-introduced into the same individual.

As used herein, the term "allogeneic" means that the material is derived from a different animal or different patient of the same species as the individual into which the material is introduced. When the genes at one or more loci are different, two or more individuals are considered allogeneic to each other. In some cases, genetic differences in allogeneic material from various individuals of the same species may be sufficient for antigen interactions to occur.

As used herein, the term "subject" refers to a mammal. The mammal may be, but is not limited to, a human, a non-human primate, a mouse, a rat, a dog, a cat, a horse, or a cow. Mammals other than human can be advantageously used as subjects representing cancer animal models. Preferably, the subject is a human.

In an embodiment, the cancer is selected from the group consisting of brain glioma, blastoma, sarcoma, basal cell carcinoma, biliary tract cancer, bladder cancer, bone cancer, brain and CNS cancer, breast cancer, peritoneal cancer, cervical cancer, choriocarcinoma, colon and rectal cancer, connective tissue cancer, cancer of digestive system, endometrial cancer, esophageal cancer, eye cancer, head and neck cancer, stomach cancer (comprising gastrointestinal cancer), glioblastoma (GBM), liver cancer, hepatoma, intraepithelial tumor, kidney cancer, larynx cancer, liver tumor, lung cancer (such as small cell lung cancer, non-small cell lung cancer, lung adenocarcinoma and squamous lung cancer), melanoma, myeloma, neuroblastoma, oral cancer (e.g., lips, tongue, mouth, and pharynx), ovarian cancer, pancreatic cancer, prostate cancer, mesothelioma, retinoblastoma, rhabdomyosarcoma, rectal cancer, cancer of respiratory system, salivary gland cancer, skin cancer, squamous cell carcinoma, stomach cancer, testicular cancer, thyroid cancer, uterine or endometrial cancer, malignant tumor of urinary system, vulval cancer, Waldenstrom macroglobulinemia, lymphoma (comprising Hodgkin's lymphoma and non-Hodgkin's lymphoma), such as B cell lymphoma (comprising low-grade/follicular non-Hodgkin's lymphoma (NHL), small lymphocytic (SL) NHL, intermediate-grade/follicular NHL, intermediate-grade diffuse NHL, high-grade immunoblastic NHL, high-grade lymphoblastic NHL, high-grade small non-cracked cell NHL, bulky disease NHL), mantle cell lymphoma, AIDS-related lymphoma, Burkitt lymphoma, diffuse large B cell lymphoma, follicular lymphoma, MALT lymphoma, marginal zone lymphoma, plasmablastic lymphoma, plasmacytoid dendritic cell tumor, etc.), leukemia (comprising acute leukemia, such as acute lymphoblastic leukemia, acute myelogenous leukemia, acute nonlymphocytic leukemia such as acute myeloblastic leukemia (comprising undifferentiated and partially differentiated), acute promyelocytic leukemia, acute myelomonocytic leukemia, acute monocytic leukemia, erythroleukemia, acute megakaryocytic leukemia; chronic leukemia such as chronic myelogenous leukemia, chronic lymphocytic leukemia, chronic monocytic leukemia; and other specific types of leukemia such as hairy cell leukemia, prolymphocytic leukemia, plasma cell leukemia, adult T-cell leukemia, eosinophilic leukemia , basophilic leukemia, etc.), blastic plasmacytoid dendritic cell tumor, malignant lymphoproliferative disease, myelodysplasia, multiple myeloma, myelodysplasia, and post-transplantation lymphoproliferative disorder (PTLD). Preferably, the diseases that can be treated with the engineered immune cells or the pharmaceutical composition of the present disclosure are selected from: leukemia, lymphoma, multiple myeloma, brain glioma, pancreatic cancer, gastric cancer and the like.

In an embodiment, the infection comprises, but is not limited to, infections caused by viruses, bacteria, fungi, and parasites.

In an embodiment, the autoimmune disease comprises, but is not limited to, type I diabetes, celiac disease, Graves disease, inflammatory bowel disease, multiple sclerosis, psoriasis, rheumatoid arthritis, Addison disease, sicca syndrome, Hashimoto thyroiditis, myasthenia gravis, vasculitis, pernicious anemia, and systemic lupus erythematosus, etc.

The present disclosure will be described in detail below with reference to the accompanying drawings and examples. It should be noted that those skilled in the art should understand that the accompanying drawings of the present disclosure and examples thereof are only for illustrative purpose, and cannot constitute any limitation to the present disclosure. The examples of the present disclosure and the features in the examples may be combined with each other without contradiction.

### Brief Description of Drawings

Figure 1 shows the expression levels of scFv in bbzi3-CAR T cells and bbz155-CAR T cells.
Figure 2 shows the killing effect of bbzi3-CAR T cells and bbz155-CAR T cells on target cells.
Figure 3 shows the cytokine release levels of bbzi3-CAR T cells and bbz155-CAR T cells.
Figure 4 shows the expression levels of scFv of CAR-T cells in which the corresponding endogenous ligands of the NK activating receptors were knocked out.
Figure 5 shows the killing effect of CAR-T cells in which the corresponding endogenous ligands of the NK activating receptors are knocked out on target cells.
Figure 6 shows the cytokine release levels of CAR-T cells in which the corresponding endogenous ligands of the NK activating receptors were knocked out.
Figure 7 shows the expression levels of scFv of bbz2B4-CAR T cells and CAR-T cells in which CD48 was knocked out.
Figure 8 shows the killing effect of bbz2B4-CAR T cells and CAR-T cells in which CD48 is knocked out on target cells.
Figure 9 shows the changes of the proliferation level of i3KO-bbz-CAR T cells and bbz-CAR T cells in mice over time.
Figure 10 shows the change in tumor burden over time in mice treated with i3KO-bbz-CAR T cells and bbz-CAR T cells.

### Detailed Description of Embodiments

The T cells used in all the examples of the present disclosure are primary human CD4+CD8+ T cells isolated from healthy donors by leukapheresis using Ficoll-Paque^{™} PREMIUM (GE Healthcare, Cat. No. 17-5442-02).

### Example 1. Preparation of CAR T cells

Sequences encoding the following proteins were synthesized and cloned into pLVX vector (Public Protein/Plasmid Library (PPL), Cat. No.: PPL00157-4a): CD8α signal peptide (SEQ ID NO: 15), anti-CD19 scFv (SEQ ID NO: 1), CD8α hinge region (SEQ ID NO: 19), CD8α transmembrane region (SEQ ID NO: 3), 4-1BB intracellular region (SEQ ID NO: 9), CD3ζ intracellular region (SEQ ID NO: 11 ) and the intracellular region of the NK activating ligand as an additional co-stimulatory domain (CD155 intracellular region (SEQ ID NO: 29) or ICAM3 intracellular region (SEQ ID NO: 27)), and the correct insertion of the target sequence was confirmed by sequencing.

Three ml Opti-MEM (Gibco, Cat. No. 31985-070) was added to a sterile tube to dilute the above plasmid, and a packaging vector psPAX2 (Addgene, Cat. No. 12260) and an envelope vector pMD2.G (Addgene, Cat. No. 12259) were added at a ratio of plasmid : viral packaging vector : viral envelope vector = 4:2:1. Then, 120 ul X-treme GENE HP DNA transfection reagent (Roche, Cat. No. 06366236001) was added, mixed immediately, incubated at room temperature for 15min, and then the plasmid/vector/transfection reagent mixture was added dropwise to the culture flask containing 293T cells. Viruses were collected at 24 hours and 48 hours, pooled, and ultracentrifuged (25000g, 4°C, 2.5 hours) to obtain concentrated lentiviruses.

T cells were activated with DynaBeads CD3/CD28 CTSTM (Gibco, Cat. No. 40203D) and cultured at 37°C and 5% CO₂ for 1 day. Then, the concentrated lentivirus was added, and after 3 days of continuous culture, T cells expressing bbzi3-CAR and bbz155-CAR were obtained. bbz-CAR T cells without additional co-stimulatory domains (i.e., CD8α signal peptide-anti-CD19 scFv-CD8α hinge region-CD8α transmembrane region-4-1BB intracellular region-CD3ζ intracellular region) and unmodified wild-type T cells (NT) were used as controls.

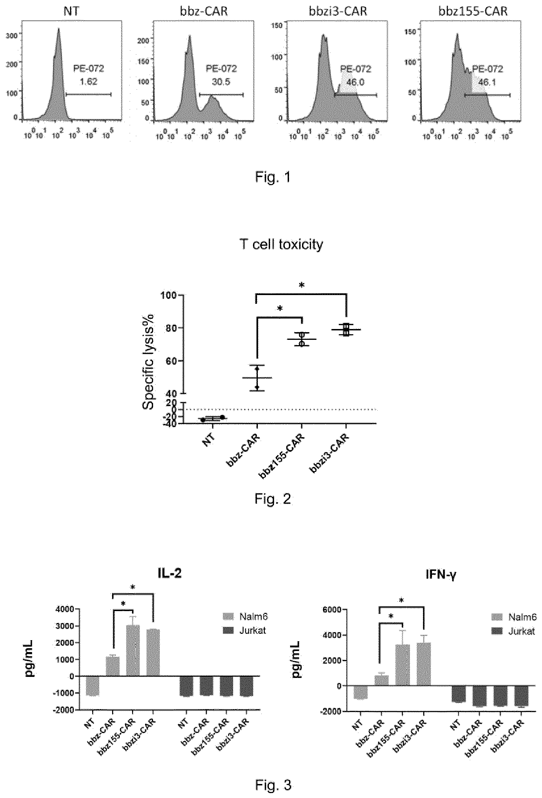

After culturing at 37°C and 5% CO₂ for 11 days, the expression of scFv on CAR-T cells was detected by flow cytometry level by using Biotin-SP (long spacer) AffiniPure Goat Anti-Mouse IgG, F(ab')₂ Fragment Specific (min X Hu, Bov, Hrs Sr Prot) (jackson immunoresearch , Cat. No. 115-065-072) as the primary antibody and APC Streptavidin (BD Pharmingen, Cat. No. 554067) or PE Streptavidin (BD Pharmingen, Cat. No. 554061) as the secondary antibody, and the results are shown in Figure 1.

It can be seen that the scFv in the CAR T cells prepared by the present disclosure is effectively expressed.

### Example 2: The killing effect of CAR T cells on target cells and the release of cytokine

### 2.1 Detection of the ability to kill target cells

Firstly, the Nalm6 target cells carrying the fluorescein gene were plated in a 96-well plate at 1×10⁴ cells/well, and then NT cells, bbz-CAR T cells, bbzi3-CAR T cells and bbz155-CAR T cells were plated into the 96-well plate at a 2:1 effector-to-target ratio (i.e. the ratio of effector T cells to target cells) for co-culture, and the fluorescence value was measured with a microplate reader after 16-18 hours. According to the calculation formula: (average fluorescence value of target cells - average fluorescence value of samples)/average fluorescence value of target cells × 100%, the killing efficiency was calculated, and the results are shown in Figure 2.

It can be seen that, compared with bbz-CAR T cells with traditional structure, the further inclusion of the intracellular region of NK activating ligand ICAM3 or CD155 as the co-stimulatory domain significantly enhances the killing ability of CAR T cells.

### 2.2 Detection of cytokine release levels

### (1) Collection of cell co-culture supernatant

Target cells Nalm6 or non-target cells Jurkat were plated in a 96-well plate at a concentration of 1×10⁵ cells/well, and then NT cells, bbz-CAR T cells, bbzi3-CAR T cells and bbz155-CAR T cells of the present disclosure were co-cultured with target cells or non-target cells at a ratio of 1:1, and the cell co-culture supernatant was collected after 18-24 hours.

### (2) ELISA detection of secretion of IL-2 and IFN-γ in the supernatant

A 96-well plate was coated with capture antibody Purified anti-human IL2 Antibody (Biolegend, Cat. No. 500302) or Purified anti-human IFN-γ Antibody (Biolegend, Cat. No. 506502) and incubated overnight at 4°C. Then the antibody solution was removed, and 250 µL of PBST (1XPBS containing 0.1% Tween) solution containing 2% BSA (sigma, Cat. No. V900933-1kg) was added, and incubated at 37°C for 2 hours. Plates were then washed 3 times with 250 µL PBST (1XPBS containing 0.1% Tween). 50 µL of cell co-culture supernatant or standards per well was added and incubated at 37 °C for 1 h, then the plate was washed 3 times with 250 µL of PBST (1XPBS with 0.1% Tween). Then 50 µL detection antibody, Anti-Interferon gamma antibody [MD-1] (Biotin) (abcam, Cat. No. ab25017) was added to each well, and incubated at 37°C for 1 hour, then the plate was washed 3 times with 250 µL PBST (1XPBS containing 0.1% Tween). Then HRP Streptavidin (Biolegend, Cat. No. 405210) was added and incubate at 37°C for 30 minutes. The supernatant was discard, and 250 µL PBST (1XPBS containing 0.1% Tween) was added for washing 5 times. 50 µL of TMB substrate solution was added to each well. Reactions were allowed to occur at room temperature in the dark for 30 minutes, after which 50 µL of 1 mol/L H₂SO₄ was added to each well to stop the reaction. Within 30 minutes of stopping the reaction, a microplate reader was used to detect the absorbance at 450nm, and the content of cytokines was calculated according to the standard curve (drawn according to the reading value and concentration of the standard), and the results are shown in Figure 3.

It can be seen that compared with bbz-CAR T cells with traditional structure, the further inclusion of the intracellular region of NK activating ligand ICAM3 or CD155 as the co-stimulatory domain significantly increases the cytokine release level of CAR T cells.

### Example 3. CART cells in which NK activating ligands have been knocked out

The corresponding NK activating ligands in bbzi3-CAR and bbz155-CAR T cells were knocked out by CRISP/Cas9 system to obtain ICAM3 knockout i3KO-bbzi3-CAR T cells and CD155 knockout 155KO-bbz155-CAR T cells. ICAM3 or CD155 in bbz-CAR T cells was also knocked out in the same way to obtain ICAM3 knockout i3KO-bbz-CAR T cells and CD155 knockout 155KO-bbz-CAR T cells.

After the CAR T cells were cultured at 37°C and 5% CO₂ for 11 days, the gene editing efficiency of CD155 and ICAM3 was detected by flow cytometry by using APC-anti human CD155 (biolegend, Cat. No. 337618) and PE-anti human ICAM3 (biolegend, Cat. No. 330005) antibodies, and the results are shown in Table 1 below.

**Table 1. Efficiency of gene editing**

| Cell name | Knockout gene | NT | Expression level after knockout |
|---|---|---|---|
| 155KO-bbz-CAR T | CD155 | 87.6% | 23.6% |
| 155KO-bbzl55-CAR T | CD155 | 87.6% | 25.8% |
| i3KO-bbz-CAR T | ICAM3 | 93.5% | 18% |
| i3KO-bbzi3-CAR T | ICAM3 | 93.5% | 19.8% |

It can be seen that various NK activating ligands were efficiently knocked out.

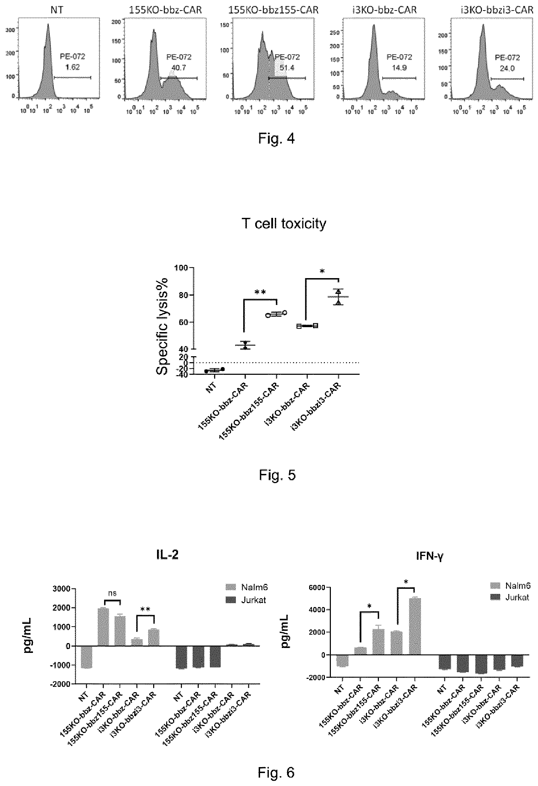

After culturing at 37°C and 5% CO₂ for 11 days, the expression of scFv on CAR T cells was detected by flow cytometry level by using Biotin-SP (long spacer) AffiniPure Goat Anti-Mouse IgG, F(ab')₂ Fragment Specific (min X Hu, Bov, Hrs Sr Prot) (jackson immunoresearch , Cat. No. 115-065-072) as the primary antibody and APC Streptavidin (BD Pharmingen, Cat. No. 554067) or PE Streptavidin (BD Pharmingen, Cat. No. 554061) as the secondary antibody, and the results are shown in FIG. 4.

It can be seen that the scFv in the CAR T cells prepared by the present disclosure can be effectively expressed.

The killing ability of the above CAR T cells on Nalm6 target cells was detected according to the method described in Example 2.1, and the results are shown in FIG. 5.

It can be seen that after the corresponding NK activating ligand is knocked out, the further inclusion of the intracellular region of NK activating ligand can significantly enhance the killing ability of CAR T cells.

According to the method described in Example 2.2, the cytokine release level after the above CAR T cells were co-cultured with Nalm6 target cells was detected, and the results are shown in FIG. 6.

It can be seen that after the corresponding NK activating ligand is knocked out, CAR T cells that further comprise the intracellular region of the NK activating ligand have a stronger ability to secrete IFN-γ. In addition, compared with the i3KO-bbz-CAR T group, the secretion level of IL-2 in the cells of the i3KO-bbzi3-CAR T group was also significantly enhanced. The above results indicate that in the context of knocking out NK activating ligands, the further inclusion of the intracellular region of the corresponding NK activating ligands can enhance the cytokine secretion ability of CAR-T cells.

### Example 4 CAR T cells expressing the intracellular segment of NK activating receptors

bbz2B4-CAR T cell was prepared according to the method in Example 1, which is different from bbz-CAR T cell only in that it further comprises the intracellular region of NK activating receptor 2B4 (SEQ ID NO: 31) as an additional co-stimulatory domain in the CAR structure. At the same time, the CD48 gene (i.e., the ligand of 2B4) in bbz-CAR T cells and bbz2B4-CAR T cells was knocked out by the CRISP/Cas9 system to obtain 48KO-bbz-CAR T cells and 48KO-bbz2B4-CAR T cells in which CD48 was knocked out.

After the CAR T cells were cultured at 37°C and 5% CO₂ for 11 days, PE-anti human CD48 (biolegend, product number 336708) was used to detect the gene editing efficiency against CD48 to confirm that CD48 was effectively knocked out. The results are shown in the following table 2.

**Table 2. Efficiency of gene editing**

| Cell name | Knockout gene | NT | Expression level after knockout |
|---|---|---|---|
| 48KO-bbz-CAR T | CD48 | 97.3% | 17.4% |
| 48KO-bbz2B4-CAR T | CD48 | 97.3% | 16.1% |

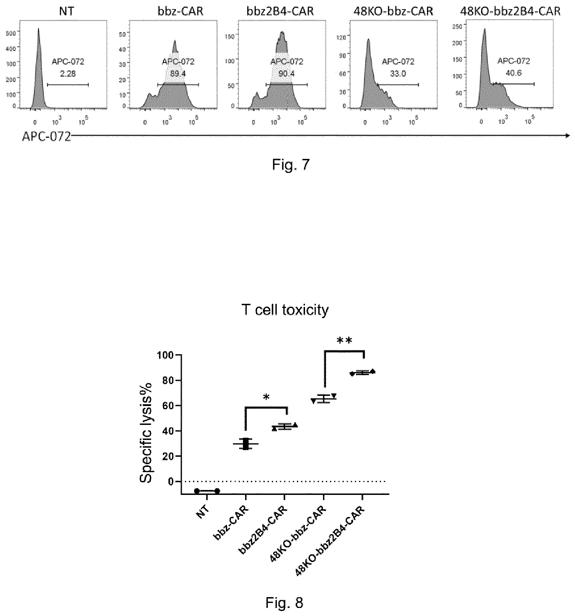

After culturing at 37°C and 5% CO₂ for 11 days, the expression of scFv on CAR T cells was detected by flow cytometry level by using Biotin-SP (long spacer) AffiniPure Goat Anti-Mouse IgG, F(ab')₂ Fragment Specific (min X Hu, Bov, Hrs Sr Prot) (jackson immunoresearch , Cat. No. 115-065-072) as the primary antibody and APC Streptavidin (BD Pharmingen, Cat. No. 554067) or PE Streptavidin (BD Pharmingen, Cat. No. 554061) as the secondary antibody, and the results are shown in Figure 7.

It can be seen that the scFv in the CAR T cells prepared by the present disclosure is effectively expressed.

The killing effect of the above-mentioned CAR T cells on Nalm6 cells was detected according to the method of 2.1 in Example 2 (Figure 8). The results showed that, whether compared with CAR T cells with traditional structure (i.e., bbz-CAR) or compared with CAR-T cells in which the NK activating ligand CD48 was knocked out (i.e., 48KO-bbz-CAR), the further inclusion of the intracellular region of NK activating ligand receptor 2B4 in the CAR structure as an additional co-stimulatory domain significantly enhances the killing ability of CAR T cells.

In addition, the applicant also unexpectedly found that compared with traditional CAR-T cells, further knocking out the NK activating ligand CD48 also significantly improves the killing ability of CAR-T cells on target cells (see Figure 8, bbz-CAR vs 48KO-bbz-CAR).

### Example 5 In vivo anti-tumor effect of CAR-T cells with knocked out of NK activating ligand

In order to further verify the effect of knocking out NK activating ligands on the function of CAR-T cells in vivo, the inventors conducted the following experiments.

Fifteen 8-week-old healthy female NCG mice were divided into three groups: NT group (negative control), bbz-CAR T group (positive control), and i3KO-bbz-CAR T group. On day 0 (D0), 5×10⁵ Raji cells were injected into the tail vein of each mouse. Three days later (D3), 2×10⁶ NT cells, bbz-CAR T cells or i3KO-bbz-CAR T cells were injected into the tail vein of each mouse according to the group. The change of Tumor burden and CAR T cell proliferation in vivo was regularly assessed by flow cytometry.

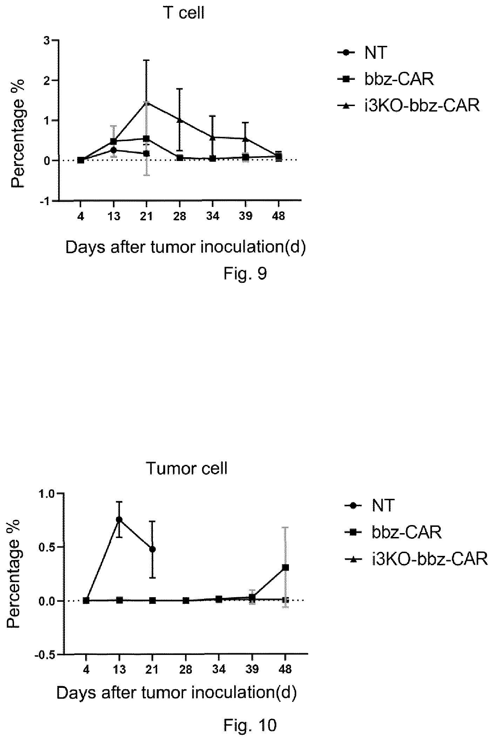

Figure 9 shows the expansion level of CAR T cells in mice over time. It can be seen that from D21 onwards, the proliferation level of i3KO-bbz-CAR T cells is much higher than that of traditional bbz-CAR T cells. Moreover, bbz-CAR T cells could not be detected in mice at D28, while i3KO-bbz-CAR T cells continued expanding in mice until D48. This indicates that knocking out the NK activating ligand ICAM3 increases the expansion level of CAR T cells in vivo and prolongs the survival time of CAR T cells in vivo.

Figure 10 shows the change of tumor burden in mice over time. It can be seen that compared with the NT group, both bbz-CAR T cells and i3KO-bbz-CAR T cells effectively inhibits the growth of tumors. But starting from D39, the tumors in the bbz-CAR T group mice began to recur, while the tumors in the i3KO-bbz-CAR T group mice continued being suppressed.

The above data show that knocking out only NK activating ligands (such as ICAM3) prolongs the persistence of CAR T cells in vivo, thereby improving the sustained killing effect on tumor cells, improving the tumor suppression effect in vivo, and increasing the survival of mice.

It should be noted that the above-mentioned are merely for preferred examples of the present disclosure and not used to limit the present disclosure. For one skilled in the art, various modifications and changes may be made to the present disclosure. Those skilled in the art should understand that any amendments, equivalent replacements, improvements, and so on, made within the spirit and principle of the present disclosure, should be covered within the scope of protection of the present disclosure.

## Claims

1. A chimeric antigen receptor comprising a ligand binding domain, a transmembrane domain, a co-stimulatory domain and an intracellular signaling domain, **characterized in that** the co-stimulatory domain comprises an intracellular region of an NK activating receptor or a ligand thereof, wherein the NK activating receptor is selected from the group consisting of 2B4, DNAM-1 and LFA-1, and the ligand of the NK activating receptor is selected from the group consisting of CD48, CD112, CD155, ICAM1, ICAM2 and ICAM3.

2. The chimeric antigen receptor according to claim 1, **characterized in that** the co-stimulatory domain comprises an intracellular region of a protein selected from the group consisting of: CD155, ICAM3, and 2B4.

3. The chimeric antigen receptor according to claim 2, **characterized in that** the CD155 intracellular region has at least 90%, 95%, 97% or 99% or 100% sequence identity to the amino acid sequence represented by SEQ ID NO: 25; the ICAM3 intracellular region has at least 90%, 95%, 97% or 99% or 100% sequence identity to the amino acid sequence represented by SEQ ID NO: 27; and the 2B4 intracellular region has at least 90%, 95%, 97% or 99% or 100% sequence identity to the amino acid sequence represented by SEQ ID NO: 27.

4. The chimeric antigen receptor according to any one of claims 1-3, **characterized in that** the co-stimulatory domain further comprises a signaling domain of a protein selected from the group consisting of: TLR1, TLR2, TLR3, TLR4, TLR5, TLR6, TLR7, TLR8, TLR9, TLR10, CARD11, CD2, CD7, CD8, CD27, CD28, CD30, CD40, CD83, CD134, CD137, CD270, CD272, CD276, CD278, CD357, DAP10, DAP12, LAT, NKG2C, SLP76, PD-1, LIGHT, TRIM, CD94, LTB, ZAP70, and a combination thereof.

5. The chimeric antigen receptor according to any one of claims 1-3, **characterized in that** the co-stimulatory domain further comprises a signaling domain of CD27, CD28, CD134, CD137 or CD278 or a combination thereof.

6. The chimeric antigen receptor according to any one of claims 1-5, **characterized in that** the ligand binding domain is selected from the group consisting of an immunoglobulin molecule, Fab, Fab', F(ab')2, Fv fragment, scFv, linear antibody, heavy chain antibody, sdAb or nanobody.

7. The chimeric antigen receptor according to any one of claims 1-6, **characterized in that** the ligand binding domain binds to one or more targets selected from the group consisting of: CD2, CD3, CD4, CD5, CD7, CD8, CD14, CD15, CD46, CD70, TSHR, CD19, CD123, CD22, BAFF-R, CD30, CD171, CS-1, CLL-1, CD33, EGFRvIII, GD2, GD3, BCMA, GPRC5D, Tn Ag, PSMA, ROR1, FLT3, FAP, TAG72, CD38, CD44v6, CEA, EPCAM, B7H3, KIT, IL-13Ra2, mesothelin, IL-1 1Ra, PSCA, PRSS21, VEGFR2, LewisY, CD24, PDGFR-β, SSEA-4, CD20, AFP, Folate receptor α, ERBB2 (Her2/neu), MUC1, EGFR, CS1, CD138, NCAM, Claudin18.2, Prostase, PAP, ELF2M, Ephrin B2, IGF-I receptor, CAIX, LMP2, gploo, bcr-abl, tyrosinase, EphA2, Fucosyl GM1, sLe, GM3, TGS5, HMWMAA, o-acetyl-GD2, Folate receptor β, TEM1/CD248, TEM7R, CLDN6, GPRC5D, CXORF61, CD97, CD179a, ALK, polysialic acid, PLAC1, GloboH, NY-BR-1, UPK2, HAVCR1, ADRB3, PANX3, GPR20, LY6K, OR51E2, TARP, WT1, NY-ESO-1, LAGE-la, MAGE-A1, legumain, HPV E6, E7, MAGE A1, ETV6-AML, sperm protein 17, XAGE1, Tie 2, MAD-CT-1, MAD-CT-2, Fos associated antigen 1, p53, p53 mutant, prostate specific protein, survivin and telomerase, PCTA-1/Galectin 8, MelanA/MART1, Ras mutant, hTERT, sarcoma translocation breakpoint, ML-IAP, ERG (TMPRSS2 ETS fusion gene), NA17, PAX3, androgen receptor, Cyclin B1, MYCN, RhoC, TRP-2, CYP1B 1, BORIS, SART3, PAX5, OY-TES 1, LCK, AKAP-4, SSX2, RAGE-1, human telomerase reverse transcriptase, RU1, RU2, intestinal tract carboxylesterase, mut hsp70-2, CD79a, CD79b, CD72, LAIR1, FCAR, LILRA2, CD300LF, CLEC12A, BST2, EMR2, LY75, GPC3, FCRL5, IGLL1, PD1, PDL1, PDL2, TGF β, APRIL, NKG2D, NKG2D ligand, and/or pathogen-specific antigen, biotinylated molecule, molecule expressed by HIV, HCV, HBV, and/or other pathogens; and/or neo-epitope or neoantigen.

8. The chimeric antigen receptor according to any one of claims 1-7, **characterized in that** the transmembrane domain is a transmembrane domain of a protein selected from the group consisting of: TCR α chain, TCR β chain, TCR γ chain, TCR δ chain, CD3 ζ subunit, CD3 ε subunit, CD3 γ subunit, CD3 δ subunit, CD45, CD4, CD5, CD8 α, CD9, CD16, CD22, CD33, CD28, CD37, CD64, CD80, CD86, CD134, CD137 and CD154.

9. The chimeric antigen receptor according to any one of claims 1-8, **characterized in that** the intracellular signaling domain is a signaling domain of a protein selected from the group consisting of: FcR γ, FcR β, CD3 γ, CD3 δ, CD3 ε, CD3 ζ, CD22, CD79a, CD79b, and CD66d.

10. A nucleic acid molecule encoding the chimeric antigen receptor according to any one of claims 1-9.

11. A vector comprising the nucleic acid molecule according to claim 10.

12. An engineered immune cell comprising the chimeric antigen receptor according to any one of claims 1-9, the nucleic acid molecule according to claim 10 or the vector according to claim 11.

13. The engineered immune cell according to claim 12, **characterized in that** an endogenous expression of the NK activating receptor or ligand thereof corresponding to the co-stimulatory domain in the engineered immune cell is suppressed or silenced.

14. An engineered immune cell, **characterized in that** an expression of an NK activating receptor or ligand thereof is suppressed or silenced, wherein the NK activating receptor is selected from the group consisting of 2B4, DNAM-1 and LFA-1, and the ligand of the NK activating receptor is selected from the group consisting of CD48, CD112, CD155, ICAM1, ICAM2 and ICAM3.

15. The engineered immune cell according to any one of claims 12-14, **characterized in that** the immune cell further comprises suppressed or silenced expression of at least one gene selected from the group consisting of: TRAC, TRBC, HLA-A, HLA-B, HLA-C, B2M, RFX5, RFXAP, RFXANK, CIITA, PD1, LAG3, TIM3, CTLA4.

16. The engineered immune cell according to any one of claims 12-15, **characterized in that** the immune cell is selected from the group consisting of a T cell, a macrophage, a dendritic cell, a monocyte, a NK cell or a NKT cell.

17. The engineered immune cell according to any one of claims 12-16, **characterized in that** the immune cell is derived from an adult stem cell, an embryonic stem cell, an umbilical cord blood stem cell, a progenitor cell, a bone marrow stem cell, an induced pluripotent stem cell, a totipotent stem cell, or a hematopoietic stem cell.

18. The engineered immune cell according to any one of claims 12-16, **characterized in that** the immune cell also expresses a chimeric antigen receptor or a recombinant T cell receptor.

19. The engineered immune cell according to claim 18, **characterized in that** the immune cell further expresses a chimeric antigen receptor comprising a ligand binding domain, a transmembrane domain, a co-stimulatory domain and intracellular signaling domain, wherein the co-stimulatory domain is selected from a signaling domain of CD27, CD28, CD134, CD137 or CD278 or a combination thereof.

20. A pharmaceutical composition comprising the engineered immune cell according to any one of claims 12-19, and one or more pharmaceutically acceptable excipients.

21. Use of the chimeric antigen receptor according to any one of claims 1-9, the nucleic acid molecule according to claim 10, the vector according to claim 11, the engineered immune cell according to any one of claims 12-19 or the pharmaceutical composition according to claim 20 in the preparation of a medicament for treating cancers, infections or autoimmune diseases.
